# EUROPÄISCHE PATENTSCHRIFT

(11) **EP 1 569 885 B1**
(45) Veröffentlichungstag und Bekanntmachung des Hinweises auf die Patenterteilung: **27.12.2006**
(21) Anmeldenummer: 03782215.2
(22) Anmeldetag: 20.11.2003
(51) Int. Cl.: C07C 45/40, C07C 45/53

(54) **IN-SITU ZERSETZUNG VON PEROXIDEN W HREND DER OZONOLYZE VON ALKENEN**
IN-SITU DECOMPOSITION OF PEROXIDES DURING THE OZONOLYSIS OF ALKENES
DECOMPOSITION I IN SITU /I DE PEROXYDES DANS L'OZONOLYSE D'ALCENES

(30) Priorität: 13.12.2002 AT 18612002
(43) Veröffentlichungstag der Anmeldung: 07.09.2005
(73) Patentinhaber: DSM Fine Chemicals Austria Nfg GmbH & Co KG, 4021 Linz (AT)
(72) Erfinder: JARY, Walther, A-4853 Steinbach am Attersee (AT); PÖCHLAUER, Peter, A-4040 Linz (AT); PERNDORFER, Eduard, A-4050 Traun (AT)
(74) Vertreter: Lindinger, Ingrid
(86) Internationale Anmeldenummer: PCT/EP2003/012995
(87) Internationale Veröffentlichungsnummer: WO 2004/054950

(56) Entgegenhaltungen:
- EP-A- 0 147 593
- DATABASE WPI Section Ch, Week 197532 Derwent Publications Ltd., London, GB; Class E19, AN 1975-53656W XP002277807 & SU 433 126 A (GILCMENOK N D), 15. Dezember 1974 (1974-12-15)

## Beschreibung

Die vorliegende Erfindung betrifft ein Verfahren zur in-situ Zersetzung von Peroxiden während der Ozonolyse von Alkenen zu den korrespondierenden Aldehyden oder Ketonen unter Verwendung eines Katalysators.

Die Ozonolyse von Olefinen liefert auf umweltfreundliche Weise Carbonylverbindungen, wie Aldehyde oder Ketone, die wertvolle Ausgangsstoffe in der präparativen, organischen Chemie darstellen.
Die Herstellung von Carbonylverbindungen aus organischen Verbindungen, die als Strukturelement eine oder mehrere C = C-Doppelbindungen im Molekül aufweisen, mittels eines zweistufigen Ozonolyse- und Reduktionsprozesses ist bekannt. Bei der Durchführung dieser Methode wird in der ersten Stufe zur Erzielung einer möglichst vollständigen Ozonisierung der Doppelbindung meistens mit einem Ozonüberschuss gearbeitet. Die in der zweiten Stufe folgende reduktive Spaltung bereitet immer wieder Schwierigkeiten, da die peroxidhaltigen Ozonisierungsprodukte instabil sind und in Abwesenheit von metallischen Hydrierkatalysatoren besonders leicht Umlagerungen oder Zersetzung erfahren, bevor sie zu den entsprechenden Carbonylverbindungen reduziert werden können. Außerdem werden bei Edelmetallkatalysatoren bei längerem Kontakt mit peroxidhaltigen Lösungen Aktivitätsverluste des Katalysators beobachtet, sodass die Lösungen bei der reduktiven Spaltung durch Hydrierung in der Regel nicht gänzlich peroxidfrei werden und neben Schwierigkeiten bei der Reindarstellung der Endprodukte auch Ausbeuteverluste und eine Explosionsgefahr hingenommen werden müssen.
Zur Vermeidung dieser Schwierigkeiten wird in US-PS-3 145 232 ein Verfahren zur Herstellung von Carbonylverbindungen empfohlen, bei der die reduktive Spaltung nach der Ozonolyse bei Temperaturen unterhalb von -40°C in Anwesenheit eines Trialkylphosphits durchgeführt wird. Neben dem apparativen Aufwand zur Herstellung der extrem tiefen Reaktionstemperaturen erfordert eine solche Reaktionsführung die Verwendung von absolut wasserfreien Lösungsmitteln, da die Trialkylphosphite in wasserhaltigen Lösungsmitteln äußerst rasch hydrolysiert werden. Außerdem bereitet die Abtrennung der freien Carbonylverbindungen von den bei der Reduktion entstehenden Phosphatestern erhebliche Schwierigkeiten.
Da nachgewiesen wurde, dass sich tiefe Reaktionstemperaturen nachteilig auf die Aktivität der eingesetzten Reduktionsmittel auswirken und deshalb Ausbeuteverluste entstehen, wird nach einem Verfahren zur Herstellung von aliphatischen, aromatischen und heteroaromatischen Aldehyden, wie es in US-PS-3 637 721 beschrieben ist, zwar die Ozonolyse der C=C-Doppelbindung bei -50°C durchgeführt, während die Reaktionstemperaturen im Verlaufe der reduktiven Spaltung der Ozonisierungsprodukte mit aromatischen oder aliphatischen Sulfiden oder Disulfiden bis auf 50°C gesteigert wird. Beim genannten Verfahren gestaltet sich aber die Abtrennung der bei der Reduktion als Begleitprodukte entstehenden Sulfoxide, beispielsweise Dimethylsulfoxid, von den als Verfahrensprodukte entstehenden Aldehyden als äußerst schwierig und ist in vielen Fällen ohne Derivatisierung der Aldehyde überhaupt undurchführbar.
In US-PS-3 705 922 oder DE-OS-2 514 001 ist schließlich die Herstellung von Carbonylverbindungen mittels eines Ozonolyse- und Reduktionsverfahrens beschrieben, bei dem die als Ausgangsmaterial dienenden ungesättigten Verbindungen mit einem Überschuss an Ozon umgesetzt und die dabei gebildeten Ozonisierungsprodukte durch katalytische Hydrierung reduktiv gespalten werden. Dabei muss überschüssiges Ozon aber vor der reduktiven Aufspaltung zum Schutz des Hydrierkatalysators vor Aktivitätsverlusten durch Spülung der Reaktionslösung mit einem Inertgas, beispielsweise mit Stickstoff, in einem eigenen Arbeitsgang wieder entfernt werden.
Zur Durchführung der Hydrierung setzt man dann dem bei der Ozonolyse gebildeten Reaktionsgemisch den Katalysator, der bevorzugt ein Edelmetallkatalysator ist, direkt zu und leitet bis zur Sättigung Wasserstoff ein.
Da Edelmetallkatalysatoren bei längerem Kontakt mit organischen Peroxiden deaktiviert werden, hängt bei den bekannten Verfahren die Ausbeute bei der Hydrierung von der Menge des jeweils eingesetzten Hydrierkatalysators ab. Wie aus einem Vergleich der Beispiele in US-PS-3 705 922 hervorgeht, nimmt die Ausbeute trotz entsprechend verlängerter Reaktionszeit um etwa 10 % ab, wenn bei gleicher Ansatzgröße anstelle von 0.5 g nur 0.2 g eines Pd/Al₂O₃-Katalysators verwendet werden. In den genannten Druckschriften finden sich aber auch keine Angaben über die Möglichkeiten zur Regenerierung oder Wiederverwendung der eingesetzten Edelmetallkatalysatoren nach Beendigung der Hydrierung.
Verfahren zur Herstellung von Carbonylverbindungen durch Ozonolyse und Reduktion, die obige Nachteile vermeiden sollen und die in technischem Maßstab durchgeführt werden, sind beispielsweise in EP-B-O 146 784 oder EP-B-O 147 593 beschrieben. Gemäß der Offenbarung dieser beiden Patentschriften werden Verbindungen, die olefinische Doppelbindungen aufweisen, in einem niedrigen aliphatischen Alkohol bei Temperaturen von -80 °C bis 20 °C mit der äquivalenten Menge Ozon umgesetzt, worauf die peroxidische Reaktionslösung in eine Suspension eines Hydrierkatalysators unter Zugabe von Wasserstoff in einer solchen Weise eingespeist wird, dass in der Reaktionsmischung eine Peroxidkonzentration von 0,1 Mol/l nicht überschritten wird. Da bei dieser Art der Reaktionsführung saure Nebenprodukte entstehen, die den Katalysator vergiften und rasch deaktivieren würden, muss der pH- Wert der Reaktionsmischung durch Zugabe einer Base kontrolliert werden.

Aufgabe der vorliegenden Erfindung war es demnach ein Verfahren zur Ozonolyse von Alkenen zu finden, bei welchem die Nachteile der bisher bekannten Verfahren vermieden werden. Insbesonders war es Aufgabe ein Verfahren zu finden, das es ermöglicht, die gewünschten Aldehyde oder Ketone in nur einem Schritt zu erhalten, wobei Probleme durch Peroxide vermieden werden.

Unerwarteterweise konnte diese Aufgabe durch ein Verfahren gelöst werden, bei welchen durch Zugabe spezieller Katalysatoren die sich während der Ozonolyse bildenden Peroxide gleich im Ozonolyseschritt quantitativ zersetzt werden.

Gegenstand der vorliegenden Erfindung ist demnach ein Verfahren zur in-situ Zersetzung von Peroxiden bei der Ozonolyse von gegebenenfalls substituierten Alkenen zu den korrespondierenden Aldehyden oder Ketonen, das dadurch gekennzeichnet ist, dass die gegebenenfalls substituierten Alkene in Wasser, einem C₁-C₄-Carbonsäureester, einer C₁-C₄-Carbonsäure, einem Wasser/C₁-C₄-Carbonsäure-Gemisch, einem C₁-C₆-Alkohol oder in einem Wasser/C₁-C₆-Alkohol-Gemisch bei einer Temperatur von -30°C bis +50°C in Gegenwart eines heterogenen, anorganischen Katalysators aus der Gruppe Iridium, Mangan, Cobalt, Silber, Gold, Palladium, Platin oder Ruthenium, der auf einem Träger aus der Gruppe Calciumcarbonat, Kohle, Titandioxid, Magnesiumoxid, Zirkonoxid, Silica oder Aluminiumoxid aufgebracht ist oder in Gegenwart eines homogenen, anorganischen Katalysators aus der Gruppe der Übergangsmetallkatalysatoren, unter gleichzeitiger Peroxidzersetzung mit Ozon umgesetzt werden, worauf nach beendeter Ozonolyse der Katalysator vom Reaktionsgemisch abgetrennt wird und die entsprechenden Aldehyde oder Ketone erhalten werden.

Bei dem erfindungsgemäßen Verfahren zur in-situ Zersetzung von Peroxiden bei der Ozonolyse werden gegebenenfalls substituierte Alkene in die entsprechenden Aldehyde oder Ketone überführt.
Geeignete Alkene sind dabei lineare, verzweigte oder cyclische Alkene mit 2-20 C-Atomen, die eine oder bis zu 6 Doppelbindungen enthalten. Cyclische Alkene können dabei auch überbrückt sein.
Beispiele für geeignete Alkene sind etwa Ethylen, Propen, 1-Buten, 2-Buten, Isobuten, 1-Penten, 2-Penten, 2-Methyl-1-buten, 2-Methyl-2-buten, 3-Methyl-1-buten, Butadien, 2-Hexen, 1-Decen, Cycloocten, Cyclooctadien, Cyclodecen, α- Terpinen, α-Pinen, u.s.w.
Die Alkene können dabei gegebenenfalls ein- oder mehrfach durch unter den Reaktionsbedingungen inerte Gruppen substituiert sein.
Geeignete Substituenten sind dabei C₆-C₁₀-Aryl oder Biaryl, wie etwa Phenyl, Biphenyl oder Naphthyl; C₆-C₁₀-Heterocyclen, wie etwa Pyridyl, Indoyl oder Chinolinyl, COOH, OH, COOR, CN, NO₂, NH₂, NHR, NR₂ oder OR, wobei R gleich C₁-C₁₀-Alkyl ist.

Bevorzugt werden lineare, verzweigte oder cyclische, nicht überbrückte Alkene mit 3-12 C-Atomen und einer oder zwei Doppelbindungen in der Alkylkette eingesetzt, die gegebenenfalls ein- oder zweifach durch COOH, COOR mit R gleich C₁-C₄-Alkyl, Phenyl oder OH substituiert sind.

Das gegebenenfalls substituierte Alken wird sodann in Wasser, einem C₁-C₄-Carbonsäureester, einem C₁-C₆-Alkohol, einer C₁-C₄-Carbonsäure oder in einem Gemisch aus Wasser und C₁-C₆-Alkohol oder Wasser und C₁-C₄-Carbonsäure gelöst.
Als C₁-C₄-Carbonsäureester eignen sich beispielsweise Ethylacetat, Butylacetat, u.s.w..
Als C₁-C₆-Alkohol kommen Methanol, Ethanol, n-Propanol, i-Propanol, n-Butanol oder Hexanol in Frage. Bevorzugt werden C₁-C₄-Alkohole und besonders bevorzugt Methanol eingesetzt.
Als C₁-C₄-Carbonsäure kommen Ameisensäure, Essigsäure, Propionsäure oder Buttersäure in Frage. Besonders bevorzugt wird Essigsäure verwendet.
Werden Wasser/Alkohol-bzw. Säure-Gemische als Lösungsmittel eingesetzt, so kann das Volumenverhältnis von Wasser zu Alkohol bzw. zu Säure zwischen 98:2 bis 2:98, bevorzugt zwischen 80:20 bis 40:60 betragen.

Anschließend wird der anorganische Katalysator zugesetzt.
Prinzipiell können heterogene oder homogene Katalysatoren verwendet werden.

Als heterogener, anorganischer Katalysator kommen Iridium, Mangan, Kobalt, Silber, Gold, Palladium, Platin oder Ruthenium in Frage. Der Katalysator befindet sich dabei auf einem geeigneten Träger aus der Gruppe Calciumcarbonat (CaCO₃), Titandioxid (TiO₂), Kohle (C), Aluminiumoxid (Al₂O₃), Magnesiumoxid (MgO), Zirkonoxid (ZrO₂), oder Silica (SiO₂).
Bevorzugte heterogene Katalysatoren sind Iridium und Silber.

Als homogene, anorganische Katalysatoren kommen Übergangsmetallkatalysatoren mit oder ohne Liganden in Frage.
Geeignete Übergangsmetalle sind dabei Co(II), Fe, Mn, Re, Ru, V, Ti, Pt, Cu, Ni und Zn. Diese Katalysatoren können gegebenenfalls in Kombination mit Liganden vorliegen. Als Liganden kommen beispielsweise Citrate, Bipyridyl, Tartrate, Phenanthroline, EDTA (Ethylendiamintetraessigsäure), DTPA (Diethylen-triamin-pentaessigsäure), Porphyrin, N,N-bis(salicyliden)(butylendiamin), Morpholine, Methylpiperazine, Piperazine, Piperidine, Pyridincarbonsäure, TMTACN (Trimethyl-triaza-cyclononan) u.s.w. in Frage.

Bevorzugt werden als homogener Katalysator Mangan(II), Mangan(III) oder ein Mangan(II)-Mangan(III)-Gemisch eingesetzt.

Die Menge an zugesetztem Katalysator beträgt 0,001 bis 30 Mol%, bezogen auf das Alken, bevorzugt 0,005 bis 20 Mol% und besonders bevorzugt 0,01 bis 2 Mol%.
Die verwendeten Katalysatoren sind kommerziell erhältlich.

Es kann jedoch auch zuerst der Katalysator vorgelegt werden und anschließend das Lösungsmittel und das Alken zugegeben werden.

Die so erhaltene Reaktionslösung wird sodann in Abhängigkeit vom gewählten Lösungsmittel auf eine Temperatur von -30°C bis +60°C, bevorzugt auf -20°C bis +45°C, gebracht, worauf ein Ozon/Sauerstoffstrom in das Reaktionsgemisch eingeleitet wird. Ozon wird dabei bevorzugt in äquimolarer Menge eingesetzt. Es kann jedoch auch ein Überschuss an Ozon eingesetzt werden.
Nach beendeter Ozonolyse wird der Katalysator durch Filtration vom Reaktionsgemisch abgetrennt.
Der Restperoxidgehalt in der so erhaltenen Reaktionslösung, die den entsprechenden Aldehyd oder das Keton enthält, kann durch das erfindungsgemäße Verfahren auf einen Wert unter 2% des insgesamt gebildeten Peroxids gesenkt werden, sodass der entsprechende Aldehyd oder das Keton einfach durch Abtrennen des Lösungsmittels oder gegebenenfalls durch geeignete Methoden, wie Extraktion, Destillation oder Kristallisation isoliert werden kann.

### Beispiel 1-5: Ozonolyse von Maleinsäure

In 200 ml Methanol/Wasser 1/1 wurden 17,4 g [150 mmol] Maleinsäure aufgelöst und ein Katalysator zugegeben (siehe Tabelle 1). Die Lösung wurde auf-15 °C temperiert und danach wurde ein Ozon/Sauerstoffstrom [60g/Nm³ Ozon] eingeleitet. Nach beendeter Ozonolyse betrug der Ozonverbrauch 149 mmol (99,3% d. theoretischen Menge). Der Katalysator wurde mittels Filtration entfernt. Die Bestimmung des Peroxidgehaltes erfolgte mittels lodometrie.

**Tabelle 1:**

| Beispiel | Katalysator | Temp. | PO**-[mmol/l] | PO-Zersetzung % |
|---|---|---|---|---|
| | [mol%] | [°C] | (% d. Th) | |
| 1 | kein Katalysator | -15 | [515] (68) | 32 |
| 2 | Co-Kat. [1]* | -15 | [420] (56) | 48 |
| 3 | Co-Kat[1] | -15 | [190] (25) | 75 |
| 4 | Mn-Kat. [1] | -15 | [65] (8,6) | 91,4 |
| 5 | Ir-Kat. [1]* | -15 | [20] (2,6) | 97,4 |

| | | | | |
|---|---|---|---|---|
| * heterogener Katalysator (Co auf Aluminiumoxid; Ir auf Calciumcarbonat) ** PO = Peroxid Gehalt | | | | |

### Beispiel 6-13: Ozonolyse von Maleinsäure

In 200 ml Wasser wurden 5,8 g [50 mmol] Maleinsäure aufgelöst und ein Ir- Katalysator auf Calciumcarbonat bzw. Ag-Katalysator auf Kohle zugegeben. Die Lösung wurde temperiert (siehe Tabelle 2) und danach wurde ein Ozon/Sauerstoffstrom [60g/Nm³ Ozon] eingeleitet. Nach beendeter Ozonolyse betrug der Ozonverbrauch 49,3 mmol (100% d. theoretischen Menge). Der Katalysator wurde mittels Filtration entfernt. Eine Bestimmung des Peroxidgehaltes erfolgte mittels lodometrie.

**Tabelle 2:**

| Beispiel | Katalysator | Temp. | PO**-[mmol/l] | PO-Zersetzung % |
|---|---|---|---|---|
| | [mol%] | [°C] | (% d. Th) | |
| 6 | 0,1 | 0 | [60] (24) | 76 |
| 7 | 0,1 | 5 | [15] (6) | 94 |
| 8 | 0,1 | 10 | [8] (3) | 92 |
| 9 | 1 (Ag/C) | 10 | [22] (9) | 91 |
| 10 | 1 | 10 | [<5] (<2) | >98 |
| 11 | 1 | 25 | [<5] (<2) | >98 |
| 12 | 1 | 30 | [<5] (<2) | >98 |
| 13 | 1 | 40 | [<5] (<2) | >98 |

| | | | | |
|---|---|---|---|---|
| ** PO = Peroxid Gehalt | | | | |

### Beispiel 14: Ozonolyse von Naphthalin

In 200 ml Essigsäure wurden 2,56 g[20 mmol] Naphthalin aufgelöst und 0,2 mmol Mn-Katalysator zugegeben. Die Lösung wurde auf 16 °C temperiert und danach wurde ein Ozon/Sauerstoffstrom [24g/Nm³ Ozon] eingeleitet. Nach beendeter Ozonolyse betrug der Ozonverbrauch 44 mmol (110% d. theoretischen Menge). Der Katalysator wurde mittels Filtration entfernt. Eine Bestimmung des Peroxidgehaltes ergab 0,1% Restperoxid in der Reaktionslösung (99,9 % Peroxidzersetzung).

### Beispiel 15-22: Ozonolyse von Cycloocten

In 200 ml Essigsäure wurden 2,2 g [53 mmol] Cycloocten aufgelöst und ein Katalysator (siehe Tabelle 3) zugegeben. Die Lösung wurde auf 16°C temperiert und danach wurde ein Ozon/Sauerstoffstrom [23,5g/Nm³ Ozon] eingeleitet. Nach beendeter Ozonolyse betrug der Ozonverbrauch 53 mmol (100% d. theoretischen Menge).

**Tabelle 3:**

| Beispiel | Katalysator | Temp. | PO**-[mmol/l] | PO-Zersetzung % |
|---|---|---|---|---|
| | [mol%] | [°C] | (% d. Th) | |
| 15 | Mn-(II)-Kat [1] | 16 | 75 (71) | 29 |
| 16 | Mn-(III)-Kat [1] | 16 | 38 (36) | 62 |
| 17 | Fe-(II)-Kat [1] | 16 | 78 (74) | 22 |
| 18 | Fe-(III)-Kat [1] | 16 | 65 (61) | 35 |
| 19 | Mn(II)-(III)-Kat [1] | 16 | 23 (21) | 77 |
| 20 | Ag/CeVOx/TiO₂ [1] | 16 | 49 (46) | 54 |
| 21 | Ag/CeVOx/SiO₂ [1] | 16 | 55 (52) | 48 |
| 22 | kein Kat | 16 | 106(100) | 0 |

### Beispiel 23-25: Ozonolyse von 1-Decen

In 200 ml Lösungsmittel (Tabelle 4) wurden 10 g [70 mmol] 1-Decen aufgelöst und 1,92 g 5% Katalysator zugegeben. Die Lösung wurde auf 16 °C temperiert und danach wurde ein Ozon/Sauerstoffstrom [60g/Nm3 Ozon] eingeleitet. Nach beendeter Ozonolyse betrug der Ozonverbrauch 70 mmol (100% d. theoretischen Menge). Der Katalysator wurde mittels Filtration entfernt.

**Tabelle 4:**

| Beispiel | Lösungsmittel [v/v] | Katalysator [mol%] | Temp [°C] | PO**-[mmol/I] (% d. Th) | PO-Zersetzung % |
|---|---|---|---|---|---|
| 23 | Essigsäure | Mn(II)-Mn(III) [0,1] | 16 | 20 (19) | 81 |
| 24 | Essigsäure/Wasser [10/1] | Mn(II)-Mn(III) [0,1] | 16 | 54 (50) | 50 |
| 25 | Essigsäure/Wasser[1/1] | Mn(II)-Mn(III) [0,1] | 16 | 12 (11) | 89 |

## Patentansprüche

1. Verfahren zur in-situ Zersetzung von Peroxiden bei der Ozonolyse von gegebenenfalls substituierten Alkenen zu den korrespondierenden Aldehyden oder Ketonen, **dadurch gekennzeichnet, dass** die gegebenenfalls substituierten Alkene in Wasser, einem C₁-C₄-Carbonsäureester, einer C₁-C₄-Carbonsäure, einem Wasser/C₁-C₄-Carbonsäure-Gemisch, einem C₁-C₆-Alkohol oder in einem Wasser/C₁-C₆-Alkohol-Gemisch bei einer Temperatur von -30°C bis +50°C in Gegenwart eines heterogenen, anorganischen Katalysators aus der Gruppe Iridium, Mangan, Cobalt, Silber, Gold, Palladium, Platin oder Ruthenium, der auf einem Träger aus der Gruppe Calciumcarbonat, Kohle, Titandioxid, Magnesiumoxid, Zirkonoxid, Silica oder Aluminiumoxid aufgebracht ist oder in Gegenwart eines homogenen, anorganischen Katalysators aus der Gruppe der Übergangsmetallkatalysatoren, unter gleichzeitiger Peroxidzersetzung mit Ozon umgesetzt wird, worauf nach beendeter Ozonolyse der Katalysator vom Reaktionsgemisch abgetrennt wird und die entsprechenden Aldehyde und Ketone erhalten werden.

2. Verfahren nach Anspruch 1, **dadurch gekennzeichnet, dass** als Alkene lineare, verzweigte oder gegebenenfalls überbrückte, cyclische Alkene mit 2 bis 20C-Atomen, die 1 bis 6 Doppelbindungen enthalten und die gegebenenfalls ein- oder mehrfach durch C₆-C₁₀-Aryl, C₆-C₁₀-Heteroaryl, COOH, OH, COOR, OR, CN oder NO₂ mit R gleich C₁-C₁₀-Alkyl substituiert sein können eingesetzt werden.

3. Verfahren nach Anspruch 1, **dadurch gekennzeichnet, dass** als homogener anorganischer Katalysator Übergangsmetalle aus der Gruppe Cobalt(II), Eisen, Mangan, Rhenium, Ruthenium, Vanadium, Titan, Platin, Cu, Ni oder Zink, gegebenenfalls in Kombination mit Liganden aus der Gruppe Citrate, Bipyridyl, Tartrate, Phenanthroline, Ethylendiamintetraessigsäure, Diethylen-triamin-pentaessigsäure, Porphyrin, N,N-bis(salicyliden)(butylendiamin), Morpholine, Methylpiperazine, Piperazine, Piperidine, Pyridincarbonsäure und Trimethyl-triaza-cyclononan eingesetzt werden.

4. Verfahren nach Anspruch 1, **dadurch gekennzeichnet, dass** bei Verwendung eines Wasser/Alkohol-bzw.Säure-Gemisches als Lösungsmittel das Volumenverhältnis von Wasser zu Alkohol bzw. zu Säure zwischen 98:2 bis 2:98 beträgt.

5. Verfahren nach Anspruch 1, **dadurch gekennzeichnet, dass** als Lösungsmittel Ethylacetat, Butylacetat, Methanol, Essigsäure, ein Methanol/Wasser-Gemisch oder ein Wasser/Essigsäure-Gemisch eingesetzt wird.

6. Verfahren nach Anspruch 1, **dadurch gekennzeichnet, dass** die Menge an zugesetztem Katalysator 0,001 bis 30Gew.% bezogen auf das Alken beträgt.

7. Verfahren nach Anspruch 1, **dadurch gekennzeichnet, dass** nach beendeter Ozonolyse der Katalysator durch Filtration vom Reaktionsgemisch abgetrennt wird und der entsprechende Aldehyd oder das Keton durch Abtrennen des Lösungsmittels, Extraktion oder Kristallisation aus dem Reaktionsgemisch isoliert wird.

## Claims

1. Process for in situ decomposition of peroxides in the course of ozonolysis of optionally substituted alkenes to the corresponding aldehydes or ketones, **characterized in that** the optionally substituted alkenes are reacted with ozone with simultaneous peroxide decomposition in water, a C₁-C₄-carboxylic ester, a C₁-C₄-carboxylic acid, a water/C₁-C₄-carboxylic acid mixture, a C₁-C₆-alcohol or in a water/C₁-C₆ alcohol mixture, at a temperature of -30°C to +50°C, in the presence of a heterogeneous inorganic catalyst from the group of iridium, manganese, cobalt, silver, gold, palladium, platinum or ruthenium which has been applied to a support from the group of calcium carbonate, carbon, titanium dioxide, magnesium oxide, zirconium oxide, silica or aluminium oxide, or in the presence of a homogeneous inorganic catalyst from the group of the transition metal catalysts, and, after ozonolysis has ended, the catalyst is then removed from the reaction mixture to obtain the particular aldehydes and ketones.

2. Process according to Claim 1, **characterized in that** the alkenes used are linear, branched or optionally bridged, cyclic alkenes which have 2 to 20 carbon atoms and contain 1 to 6 double bonds and which may optionally be mono- or polysubstituted by C₆-C₁₀-aryl, C₆-C₁₀-heteroaryl, COOH, OH, COOR, OR, CN or NO₂, where R is C₁-C₁₀-alkyl.

3. Process according to Claim 1, **characterized in that** the homogeneous inorganic catalyst used is transition metals from the group of cobalt(II), iron, manganese, rhenium, ruthenium, vanadium, titanium, platinum, copper, nickel or zinc, optionally in combination with ligands from the group of citrates, bipyridyl, tartrates, phenanthrolines, ethylenediaminetetraacetic acid, diethylenetriaminepentaacetic acid, porphyrine, N,N-bis(salicylidene)(butylenediamine), morpholines, methylpiperazines, piperazines, piperidines, pyridinecarboxylic acid and trimethyltriazacyclononane.

4. Process according to Claim 1, **characterized in that**, in the case of the use of a water/alcohol or acid mixture as the solvent, the volume ratio of water to alcohol or to acid is between 98:2 and 2:98.

5. Process according to Claim 1, **characterized in that** the solvent used is ethyl acetate, butyl acetate, methanol, acetic acid, a methanol/water mixture or a water/acetic acid mixture.

6. Process according to Claim 1, **characterized in that** the amount of added catalyst is 0.001 to 30% by weight based on the alkene.

7. Process according to Claim 1, **characterized in that**, after ozonolysis has ended, the catalyst is removed from the reaction mixture by filtration and the particular aldehyde or the ketone is isolated from the reaction mixture by removing the solvent, extracting or crystallizing.

## Revendications

1. Procédé pour la décomposition in situ de peroxydes dans l'ozonolyse d'alcènes éventuellement substitués en les aldéhydes correspondants ou les cétones correspondantes, **caractérisé en ce qu'**on fait réagir avec de l'ozone les alcènes éventuellement substitués dans de l'eau, un ester d'acide carboxylique en C₁-C₄, un acide carboxylique en C₁-C₄, un mélange d'eau et d'acide carboxylique en C₁-C₄, un alcool en C₁-C₆ ou dans un mélange d'eau et d'alcool en C₁-C₆, à une température de -30 °C à +50 °C, en présence d'un catalyseur inorganique hétérogène choisi dans le groupe constitué par l'iridium, le manganèse, le cobalt, l'argent, l'or, le palladium, le platine et le ruthénium, qui est appliqué sur un support choisi dans le groupe constitué par le carbonate de calcium, le charbon, le dioxyde de titane, l'oxyde de magnésium, l'oxyde de zirconium, la silice et l'oxyde d'aluminium, ou en présence d'un catalyseur inorganique homogène choisi dans le groupe des catalyseurs à métaux de transition, avec décomposition simultanée des peroxydes, à la suite de quoi, une fois terminée l'ozonolyse, on sépare le catalyseur du mélange réactionnel et on obtient les aldéhydes correspondants et les cétones correspondantes.

2. Procédé selon la revendication 1, **caractérisé en ce qu'**on utilise en tant qu'alcènes des alcènes linéaires, ramifiés ou cycliques éventuellement pontés, ayant de 2 à 20 atomes de carbone, comportant de 1 à 6 doubles liaisons et qui peuvent éventuellement être une ou plusieurs fois substitués par aryle en C₆-C₁₀, hétéroaryle en C₆-C₁₀, COOH, OH, COOR, OR, CN ou NO₂, R étant un groupe alkyle en C₁-C₁₀.

3. Procédé selon la revendication 1, **caractérisé en ce qu'**on utilise en tant que catalyseur inorganique homogène des métaux de transition choisis dans le groupe constitué par le cobalt-(II), le fer, le manganèse, le rhénium, le ruthénium, le vanadium, le titane, le platine, le cuivre, le nickel et le zinc, éventuellement en association avec des ligands choisis parmi les citrates, le bipyridyle, les tartrates, les phénanthrolines, l'acide éthylènediaminetétraacétique, l'acide diéthylènetriaminepentaacétique, la porphyrine, la N,N-bis(salicylidène)(butylènediamine), les morpholines, les méthylpipérazines, les pipérazines, les pipéridines, l'acide pyridinecarboxylique et le triméthyl-triazacyclononane.

4. Procédé selon la revendication 1, **caractérisé en ce que** lorsqu'on utilise comme solvant un mélange eau/alcool ou acide, le rapport en volume de l'eau à l'alcool ou à l'acide est compris entre 98:2 et 2:98.

5. Procédé selon la revendication 1, **caractérisé en ce qu'**on utilise comme solvant l'acétate d'éthyle, l'acétate de butyle, le méthanol, l'acide acétique, un mélange de méthanol et d'eau ou un mélange d'eau et d'acide acétique.

6. Procédé selon la revendication 1, **caractérisé en ce que** la quantité de catalyseur ajouté va de 0,001 à 30 % en poids, par rapport à l'alcène.

7. Procédé selon la revendication 1, **caractérisé en ce qu'**une fois terminée l'ozonolyse on sépare le catalyseur par filtration du mélange réactionnel et on isole à partir du mélange réactionnel l'aldéhyde correspondant ou la cétone correspondante par séparation du solvant, extraction ou cristallisation.
